# EUROPEAN PATENT SPECIFICATION

(11) **EP 2 732 881 B1**
(45) Date of publication and mention of the grant of the patent: **06.05.2015**
(21) Application number: 12192597.8
(22) Date of filing: 14.11.2012
(51) Int. Cl.: B05B 7/00, B05B 9/04, B65D 83/14, A61M 11/00, B05B 7/24, B05B 15/04

(54) **Micro nebulizer for nasal cleaning or treatment**
Mikroinhalator für Nasenreinigung und -behandlung
Micro-nébuliseur pour le nettoyage ou le traitement du nez

(43) Date of publication of application: 21.05.2014
(73) Proprietor: Air Liquide Medical Systems S.p.A., 20148 Milano (IT)
(72) Inventor: Alberici, Luca, 25128 Brescia (IT); Sandoni, Giuseppe, 25124 Brescia (IT)
(74) Representative: Pittis, Olivier

(56) References cited:
- EP-A1- 1 044 699
- WO-A1-2007/010579
- WO-A1-2009/128109
- DE-U1-202007 017 437
- US-A- 5 505 193

## Description

The present invention concerns a nasal nebulizer for delivering a nebulized solution, such as a drug-containing liquid or solution, to the nasal cavities of a user, especially for nasal washing, cleaning, treatment or similar.

Nasal micro-nebulizers, also called nasal washing devices or nasal nebulizers, are used for nebulizing a liquid solution that is used for flushing and cleaning the nasal cavities of a user and, when a drug is added to the washing solution, for both cleaning and treating the upper airways of a user, i.e. the nasal regions of the user, in particular the interior of the nostrils.

A known nebulizer of this kind using drug or physiological solutions is commercialized by AIR LIQUIDE MEDICAL SYSTEMS and available on the market under the trademark Rinowash^{™}. It is particularly suitable for treating medical conditions or pathologies affecting the upper airways of a person, especially for treating rhinitis, rhinosinusitis, nasal polyposis, adenoiditis and tubotympanitis. It produces solution particles greater than 10 µm and acts only on the upper airways.

Such a nebulizer is also described by EP-A-1044699.

Generally speaking, the nebulized solution can typically be water, a hypertonic solution, or a medicine or drug-containing solution.

With the current nasal washing devices or nasal nebulizers, a certain amount of said liquid solution cannot be nebulised and is wasted or lost in the device, i.e. lost in the tank and/or wasted in other internal parts of the device.

The wasted amount of liquid solution is typically of about 1 to 1,5 ml, depending on the device and its design.

Those amounts of wasted solution can represent however up to about 10% of the total amount of liquid contained in the filled tank of the nasal nebulizer as the maximum capacity of a tank is of about 15 ml, due to size and design constraints.

In the case where the solution is water or a hypertonic solution, wasting 1,5 ml of 15 ml can be considered as reasonable and not a problem as those solutions are not expensive and are used in significant quantities for cleaning the nasal cavities of the user.

In contrast, when the solution contains an expensive medicine, drug or medical composition that should be delivered only in small amount to the nasal cavities, for example according to a given posology fixed by a physician or similar, the loss of up to 1,5 ml of solution is a real problem. For instance, if a physician prescribes a nasal treatment based on the administration of 2 ml of a nebulized drug-containing solution, then wasting 1 ml or 1,5 ml of these 2 ml is not conceivable as the treatment would not be efficient enough due to the loss of 50% or even more of the drug solution.

Simply reducing the volume of the tank of the nebulizer is not possible as during the course of a treatment, it happens that the first part of the treatment is done with expensive drug-solution administrated in small quantities, i.e. less than 2 or 3 ml, whereas the second part of the treatment is done with non-expensive solution, such as water or a hypertonic solution, that should be administrated in higher amounts, such as up to 15 ml, for cleaning and washing the nostril cavities.

In other words, the tank should permit efficient delivery of nebule volumes ranging from less than 2 or 3 ml up to 15 ml, so as to satisfy both requirements with a single device. Reducing the volume of the tank is not a satisfactory solution.

The problem to be solved is hence to be able to propose an improved nasal nebulizer allowing the administration of small or of high amounts of a nebulized solution, depending on the physician prescription, while minimizing the loss of said solution, especially when the solution is a drug-containing solution or similar.

The solution of the present invention is a nasal nebulizer comprising :
- a base part with a gas inlet and an internal gas passage,
- a main tank structure carried by the base part and comprising an internal compartment, and
- an internal conduct being in fluid communication with the internal gas passage of the base part, and projecting upwardly into the internal compartment of the main tank structure,
**characterized in that** it further comprises a removable mini tank structure arranged into the main tank structure and comprising a lower opening, said internal conduct projecting upwardly through said lower opening of the mini tank structure.

Depending on the considered embodiment, the nasal nebulizer device of the present invention can comprise one or several of the following features:
- the mini tank structure has a cup-like shape.
- the mini tank structure comprises an internal chamber having a reduced volume that is less than the volume of the internal compartment of the main tank structure.
- the mini tank structure comprises an internal chamber having a reduced volume that is less than 5 ml, preferably of 3 ml or less, and the internal compartment of the main tank structure has an internal volume of that is of at least 10 ml, preferably of 15 ml or more.
- the removable mini tank structure comprises a lower opening comprising a gasket element.
- the removable mini tank structure comprises connecting parts arranged for holding the mini tank structure in a predefined position into the main tank.
- the removable mini tank structure comprises connecting parts act as abutments cooperating with the circular end surface of the main tank structure so as to maintain the mini tank structure into the main tank in said predefined position, preferably the connecting parts comprise an external collar projecting outwardly.
- the removable mini tank structure, the main tank structure and the internal conduct projecting upwardly into the mini tank structure are coaxially arranged.
- it further comprises a mini internal cylinder arranged so as to form a sleeve around the internal conduct of the main tank structure, said mini internal cylinder comprising a skirt-like peripheral wall projecting downwardly into the mini tank structure toward the bottom surface of said mini tank structure and said mini internal cylinder being radially separated from the internal conduct by an intermediary spacing.
- it further comprises an inner bell-shaped nozzle and an upper part with a bell-shaped portion coaxially arranged on the mini internal cylinder.
- the inner bell-shaped nozzle, the upper part having a bell-shaped portion and the mini internal cylinder are removable/detachable.
- the internal conduct, the external peripheral wall and the bottom surface forming the main tank structure are made as one piece, for example by moulding.

Further, the invention also concerns a kit comprising :
- a nasal nebulizer according to the present invention, i.e. as described above, and
- at least one internal cylinder part conceived and designed for being arranged so as to form a sleeve around the internal conduct of the main tank structure, said internal cylinder part comprising a skirt-like peripheral wall projecting downwardly into the main tank structure toward the bottom surface of the storage compartment of said main tank structure, and said internal cylinder part being radially separated from the internal conduct by an intermediary spacing .

Optionally, the internal cylinder part and the internal conduct are made as one piece or are integral, i.e. not detachable one from the other.

The present invention will be better understood thanks to the following description made in reference to the accompanying drawings among which:
- Figure 1 represents the different elements of a nasal nebulizer according to an embodiment of the present invention,
- Figure 2 represent a lateral view of the nebulizer of Figure 1,
- Figure 3 represents an embodiment of a valve-based pressure-control system useable for controlling the airflow in the nebulizer of Figure 1,
- Figure 4 represents an embodiment of a main tank structure for storing a liquid solution in the nebulizer of Figure 1,
- Figure 5 represents the main tank structure of Figure 4 equipped with a cylinder part and an inner bell-shaped nozzle in the nebulizer of Figure 1,
- Figure 6 represents the main tank structure of Figure 5 further equipped with an upper part having a bell-shaped portion,
- Figure 7 represents an embodiment of a mini tank structure according to the present invention and useable in the nebulizer of Figure 1, and
- Figure 8 represents the mini tank structure of Figure 7 positioned in the main tank structure of the nebulizer of Figure 1.

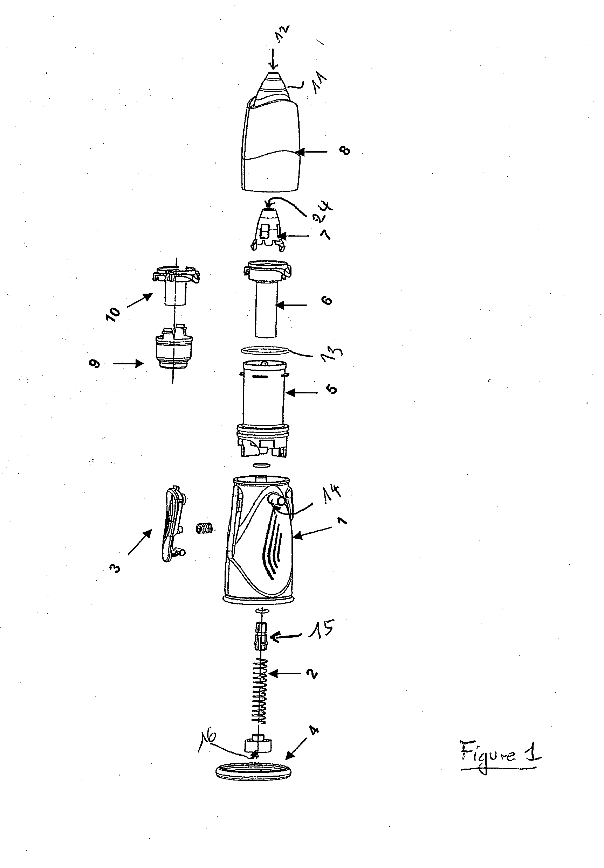

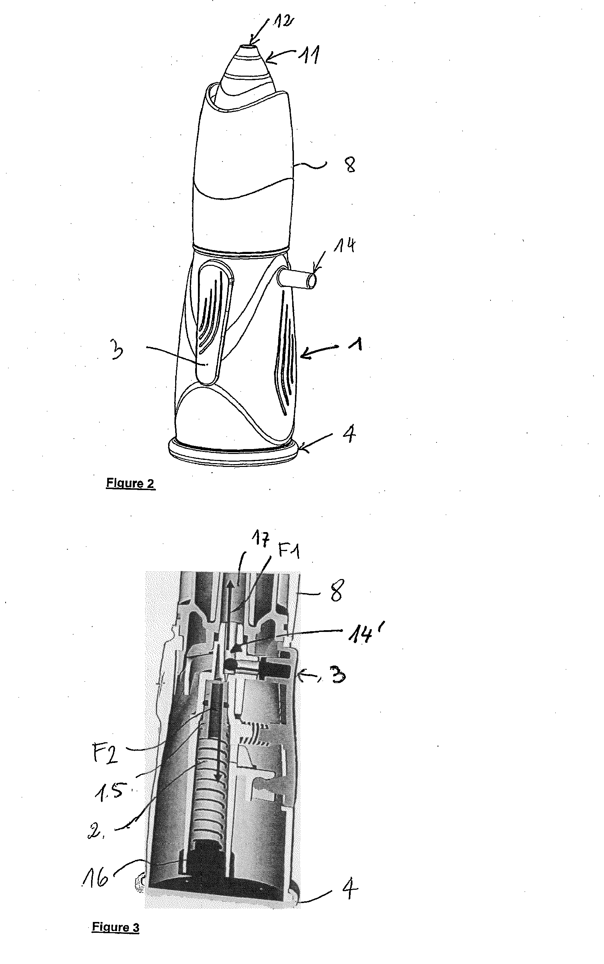

As shown in Figures 1 and 2, this embodiment of a nasal wash device or nasal nebulizer according to the present invention comprises a base part 1, a main tank structure 5 for storing a liquid solution, an internal cylinder part 6, an inner bell-shaped nozzle 7 and an upper part 8 terminated by a bell-shaped portion 11, which is inserted into the nostrils of the patient during treatment for delivering the nebulized solution through a delivery outlet or orifice 12.

Generally speaking, the base part 1, the main tank structure 5, the internal cylinder part 6, the inner bell-shaped nozzle 7, the upper part 8 and the bell-shaped portion 11 are arranged so as to be coaxial.

Further, the main tank structure 5, the internal cylinder part 6, the inner bell-shaped nozzle 7, the upper part 8 and the bell-shaped portion 11 are fit together in a removable or detachable way. In other words, they can be connected or disconnected one from the other upon user's wishes.

The main tank structure 5 where the liquid solution is stored comprises a rigid shell or wall and has an internal volume of the solution of typically more or less 15 ml.

The liquid tightness between the main tank structure 5 and the upper part 8 is ensured by means of a gasket 13 or similar made of a soft material, such as rubber or similar, thereby preventing liquid leakages.

The base 1 of the nebulizer is the part that the patient holds in hand during the treatment and the liquid solution delivery.

A flap cap 4 covers, i.e. closes, the base 1 which has a general tubular form, such as a cylindrical, quasi-cylindrical or tronconical shape. The flap cap 4 constitutes also a flat surface allowing the nebulizer to be put and stored in vertical position on a support, such as a table or similar.

For maintaining a constant delivery pressure of the nebulized liquid, at the outlet 12 of the device, the nebulizer comprises the valve-based pressure-control system described by EP-A-1044699. This prevents any fluid overpressures that could otherwise injure the patient.

Said valve-based pressure-control system is shown in Figure 3 and works as follows:
When a user actuates the press-button 3 arranged on the base 1, a flow of pressured air delivered by a compressor unit (not shown), enters into the base 1 through the inlet conduct 14 and is directed (cf. arrow F1) towards tank 5, via the internal conduct 17, for operating the nebulisation of the liquid solution contained therein as explained below.

When the pressure of the airflow exceeds a predetermined value depending on the resistance of the spring 2, said pressure acts on a mobile gasket element 15, arranged in a gas passage located between the spring 2 and the inlet conduct 14, so as to push it 15 downwardly as well as the spring 2 (cf. arrow F2) thereby breaking the tightness that is normally ensured by the gasket element 15 so that the overpressure is released at the bottom of the base 1 part, through an overpressure outlet 16.

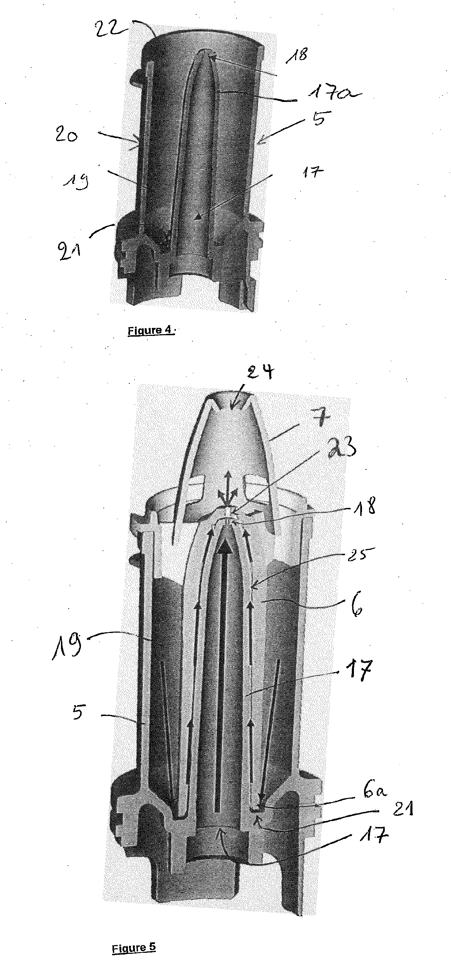

As shown in Figure 4, the main tank 5, where the liquid solution is stored, comprises a storage compartment 19 having a volume of about 15 ml for storing the liquid solution to be nebulized. Said storage compartment 19 is internally delimited by the internal conduct 17 and outwardly delimited by an external peripheral wall 20, said internal conduct 17 and said external peripheral wall 20 been linked by a bottom surface 21 forming the bottom of the storage compartment 19.

The external peripheral wall 20 has a general cylindrical-shape.

The internal conduct 17 comprises a restricted outlet orifice 18 situated in the vicinity of the upper end 22 of said storage compartment 19. In other words, the outlet orifice 18 has an internal section tapering upwardly, i.e. the internal section of the internal conduct 17 decreases progressively towards the outlet orifice 18, in the vicinity of said outlet orifice 18.

Further, the upper end 22 of said storage compartment 19 is open and has a generally cylindrical shape, i.e. a circular section.

The internal conduct 17, the external peripheral wall 20 and the bottom surface 21 linking them are made as one piece, for example by moulding. Preferably, there are made of polymer or similar, such as plastic material.

The flow of pressured air circulates through the internal conduct 17 and is delivered through the outlet orifice 18 situated at the upper end of the internal conduct 17.

Figure 5 shows the internal cylinder element 6 mounted on the tank 5 so as to form a sleeve around the internal conduct 17 of tank 5.

The first outlet orifice 18 located at the upper end of the internal conduct 17 of tank 5 and the second outlet orifice 23 located at the upper end of the internal cylinder 6 are aligned, i.e. coaxially arranged.

Further, the inner bell-shaped nozzle 7 is arranged on the internal cylinder element 6 so as to cap it and further so that the nozzle orifice 24 is also coaxially arranged with the first and second outlet orifices 18, 23.

In this way, the airflow delivered by the first outlet orifice 18 can subsequently pass through the second outlet orifice 23 located at the upper end of the internal cylinder 6 and successively through the nozzle orifice 24 located at the upper end of the inner bell-shaped nozzle 7.

Preferably, the inner bell-shaped nozzle 7 and the internal cylinder element 6 are also made of polymer or similar, such as plastic material.

In order to obtain a suction effect, i.e. a Venturi effect, allowing the liquid solution present in the tank 5 to be sucked out of the internal storage compartment or chamber 19, it is mandatory that a little spacing 25, i.e. of between about 0.2 and 5 mm, is maintained between the external surface of the internal conduct 17 of tank 5, and the internal surface of the internal cylinder element 6 as shown in Figure 5.

Such a little spacing 25 forms a circular hollow column or gap through which the liquid solution sucked out of the storage compartment 19 of the tank 5 can circulated, due to the suction force caused by the airflow conveyed by internal conduct 17 and successively passing through the first outlet orifice 18, the second outlet orifice 23 and the nozzle orifice 24.

Actually, when reaching the top of the tank 5, i.e. the region of the first outlet orifice 18 and second outlet orifice 23, the pressure of the airflow passing through the first outlet orifice 18 and the second outlet orifice 23, decreases and such a pressure drop involves a suction force, i.e. a depression, that sucks the liquid solution out of the tank reservoir 5 and through the spacing 25 to the region of the first outlet orifice 18 and second outlet orifice 23.

When the liquid solution reaches said outlet orifice 18, 23, a sudden increase of pressure occurs, which blows the liquid into fine liquid particles thereby creating a nebulised stream comprising the nebulised particles of liquid carried by the airflow, i.e. a mist.

As shown in Figure 5, the peripheral wall of the internal cylinder element 6 forms a peripheral skirt which is configured and sized so as to have an open end 6a located very close to, i.e. in the vicinity of, the bottom surface 21 of the storage compartment 19, i.e. the bottom surface 21 of the tank 5. In other words, the height of the internal cylinder element 6 more or less equals the height of the of the storage compartment 19 of the tank 5.

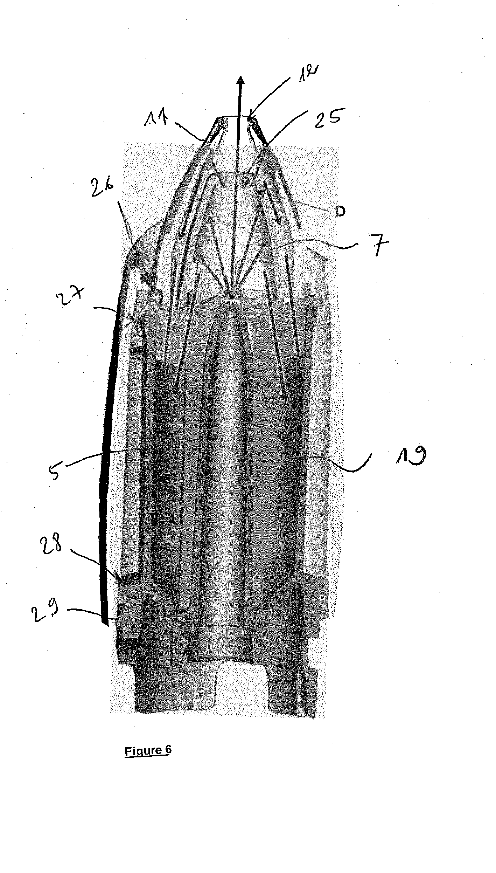

As shown in Figure 6, said nebulisation mist travels upwardly into the inner bell-shaped nozzle 7.

A part of said nebulisation mist exits the inner bell-shaped nozzle 7 through the outlet 25, while the rest impacts the inside walls of said inner bell-shaped nozzle 7 and is recovered into tank 5.

The part of nebulisation mist that exits the inner bell-shaped nozzle 7, passes through the upper part 8 terminated by the bell-shaped portion 11 and is subsequently delivered to the nostril of a patient by passing through the delivery orifice 12.

Preferably, the upper part 8 terminated by the bell-shaped portion 11 which covers and forms a sleeve around the inner bell-shaped nozzle 7 as well as around the internal cylinder element 6, is fixed to the main tank structure 5, for example it "clips" on it as shown in Figure 6.

Said upper part 8 is detachably mounted on the main tank structure 5 and can be maintained thereon thanks to abutments 26, 28, 29 or similar.

Advantageously, the upper part 8 terminated by the bell-shaped portion 11 is also made of polymer or similar, such as plastic material.

The bell-shaped portion 11 forming the upper part 8 can be made of polymeric material, such as elastomer, rubber, silicone or similar, so as to increase the comfort for the patient.

A part of said nebulisation mist exits the upper part 8 through the delivery outlet 12 and reaches the nostril. The rest of the mist, here again, impacts the inside walls of the upper part 8 and is subsequently recovered and sent back to tank 5, as illustrated in Figure 6 (see arrows), under the effects of gravity and through the upper end of tank 5 which is widely open, as already mentioned.

Preferably, the opening 23 of the inner bell is designed smaller than the opening of outer bell 24 in order to keep the nebulizer cleaner, i.e., without drops of drugs that flows on the walls of the outer bell and then go outside. This is important for reducing the drug residual.

Such an arrangement avoids or minimizes the loss of solution, especially when it contains a drug substance. However, with such an arrangement, a non-negligible amount of liquid drug solution is unfortunately lost inside the main tank structure 5.

Indeed, some of the solution remains stuck to the walls of the tank structure 5 in the form of droplets, whereas another part of the solution always stays at the bottom of the storage compartment or chamber 19 of the main tank structure 5 as it can not be draw out of the tank 5 because it represents too-small an amount for activating the Venturi effect, i.e. the suction effect.

As already explained, the total amount of lost liquid solution can be of about 1 to 1,5 ml, which represents up to about 10% of the overall capacity of the tank chamber 19 and up to 50% or even more of some posologies defined by a physician (for instance 2 ml), which is clearly a huge drawback when the liquid solution contained an expensive drug and/or when the posology fixed by the physician has to be carefully observed.

The present invention proposes a solution for solving this problem, i.e. for avoiding and at least minimizing those losses of liquid solution in the main tank structure 5, especially when the liquid solution contains an expensive drug and/or when such a drug has to be delivered in small quantities according to a particular posology.

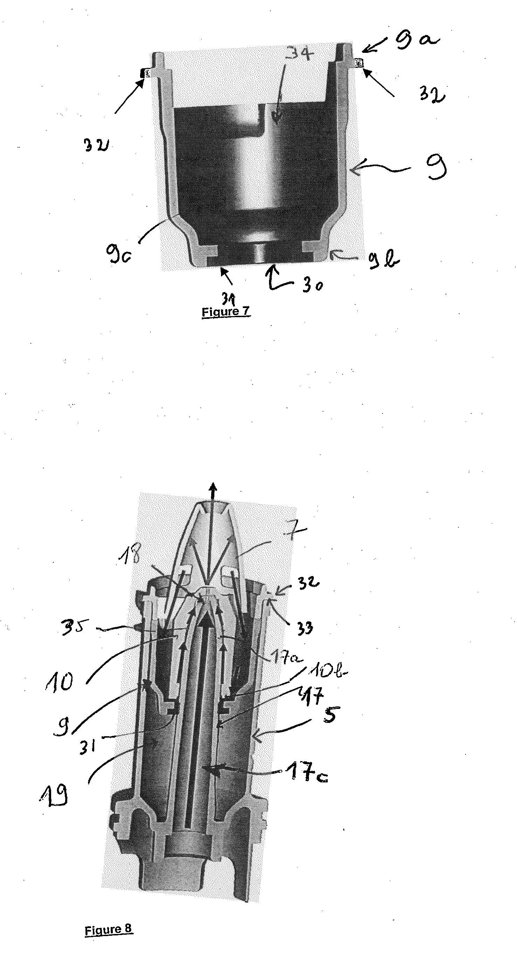

More precisely, according to the present invention a mini tank structure 9 as shown in Figure 7, is inserted into the main tank structure 5 and a mini internal cylinder 10 replaces the internal cylinder element 6 as shown in Figures 1 and 8.

As shown in Figure 7, the mini tank structure 9 as a general cup-like shape with a circular section. The mini tank structure 9 is open at its upper end 9a and its lower end 9b.

The mini tank structure 9 has a smaller volume than the main tank structure 5 and is hence better designed for receiving drug solutions.

When this part is assembled, an internal cylinder 10 allows the nebulisation of the drug, allowing the patient a normal use of the nasal wash.

The mini tank structure 9 can be positioned inside the main tank structure 5 and around the internal conduct 17 as shown in Figure 8, thereby reducing the overall volume of the reservoir or tank 5 that receives the liquid solution.

When the mini tank structure 9 is inserted on the internal conduct 17, said internal conduct 17 passes through the lower opening 30 located at the lower end 9b of the mini tank structure 9, and projects upwardly.

The mini tank structure 9 can be used for modifying the capacity of the main tank 5 in a temporary way. That is to say that it reduces the liquid reservoir capacity only for the duration of the treatment with a given drug solution, and is removable once the treatment is over.

The mini tank structure 9 is configured and designed to fit inside the main tank 5 and to generally match the internal shape of the internal walls of the main tank 5 in its upper areas.

In order to ensure a good liquid tightness, a gasket element 31 (i.e. a joint), such as an O-ring or the like, is arranged in the lower opening 30, i.e. the gasket element 31 is "sandwiched" between the mini tank structure 9 located in the lower opening 30 and the peripheral surface of the internal conduct 17. This prevents the drug solution from leaking in the main tank 5 situated under the mini tank 9 as, for limiting the drug losses, the liquid solution must stay only in the mini tank 9 and should not be able to drop in the bottom portion of the main tank 5.

At its upper end 9a, the mini tank structure 9 comprises some connecting parts 32 (see Fig. 7), such as an external collar projecting outwardly or the like, for holding the mini tank structure 9 in a desired position into the main tank 5. Those connecting parts 32 act as abutments cooperating with the circular end surface 33 of the main tank structure 5 so as to maintain/hold the mini tank structure 9 into the main tank 5.

For ensuring an efficient suction of the liquid and its subsequent nebulisation, it is mandatory to replace the internal cylinder 6 by a mini internal cylinder 10 also positioned on the top of the internal conduct 17, so as to form a sleeve around its end portion 17a carrying the outlet 18, said mini internal cylinder 10 projecting downwardly in the direction of the bottom 9c of the mini tank 9. In other words, the mini internal cylinder 10 has a smaller size than the internal cylinder 6 so as to be able to cooperate with the mini tank structure 9 and the internal conduct 17.

More precisely, the mini internal cylinder 10 has general-shape and functions that are the same as the ones of the internal cylinder 6 (see above for details). However, the peripheral wall of the mini internal cylinder 10 that forms a skirt-like structure has a height/length that is limited compared to the one of the internal cylinder 6, i. e. its height/length is chosen so that the lower end 10b of the mini internal cylinder 10 matches the internal bottom surface 9c of the mini tank structure 9.

Further, for ensuring an efficient suction of the liquid stored in the mini tank 9, for example a drug-containing solution, the mini internal cylinder 10 is radially separated from the internal conduct 17 by an intermediary spacing 35 having a small dimension thereby allowing the liquid to circulate upwardly in said spacing intermediary 35 upon the effect of the venturi or suction forces created by the airflow under pressure circulating inside 17c the internal conduct 17 and subsequently delivered by the outlet 18 at the end of the internal conduct 17, as explained above and shown in Figure 8.

Generally speaking, when a user has to clean his nasal cavities with a non-expensive or non-therapeutically active liquid solution, such as water for example, he can use the device in the configuration shown in Figures 1 to 6. In that case, the internal compartment 19 of the main tank 5 is filled with the solution, such as water, for instance 10 to 20 ml of solution, typically more or less 15 ml, as it is not important if 1 or 1,5 ml of solution are lost and wasted in the device during its utilization.

However, when the solution is or contains an expensive product and/or a therapeutically active product that should be administrated according to a specific and precise posology, such as a drug for instance, the user will have to insert the mini tank structure 9 having the cup-like form as shown in Figure 7, into the main tank 5 so as to limit its volume to less than 5 ml for example, typically to 2,5 ml or less, and further replace the internal conduct 17 by the mini internal cylinder 10, and put back the other elements of the system, i.e. the inner bell-shaped nozzle 7 and the upper part 8 terminated by a bell-shaped portion 11, which caps the inner bell-shaped nozzle 7, and which is eventually inserted into one or the nostrils of the patient, during treatment, for delivering the desired nebulized solution through the delivery outlet or orifices 12.

In the latter case, the internal compartment 19 of the main tank 5 is not filled with the expensive solution or similar, such as a drug-containing solution, as the solution is stored only in the mini tank structure 9 which has a reduced size and consequently exhibits smaller surfaces where liquid droplets can adhere and be lost. The same effect is obtained in using the mini internal cylinder 10 which is smaller than the internal conduct 17. In that way, the amount of solution lost during a treatment can be divided by at least 3 or 4 thereby minimizing the solution losses.

## Claims

1. Nasal nebulizer comprising :
- a base part (1) with a gas inlet (14) and an internal gas passage (14'),
- a main tank structure (5) carried by the base part (1) and comprising an internal compartment (19), and
- an internal conduct (17) being in fluid communication with the internal gas passage (14') of the base part (1), and projecting upwardly into the internal compartment (19) of the main tank structure (5),
**characterized in that** it further comprises a removable mini tank structure (9) arranged into the main tank structure (5) and comprising a lower opening (30), said internal conduct (17) projecting upwardly through said lower opening (30) of the mini tank structure (9).

2. Nasal nebulizer according to the previous Claim, **characterized in that** the mini tank structure (9) has a cup-like shape.

3. Nasal nebulizer according to one of the previous Claims, **characterized in that** the mini tank structure (9) comprises an internal chamber (34) having a reduced volume that is less than the volume of the internal compartment (19) of the main tank structure (5).

4. Nasal nebulizer according to one of the previous Claims, **characterized in that** :
- the mini tank structure (9) comprises an internal chamber (34) having a reduced volume that is less than 5 ml, preferably of 3 ml or less, and
- the internal compartment (19) of the main tank structure (5) has an internal volume of that is of at least 10 ml, preferably of 15 ml or more.

5. Nasal nebulizer according to one of the previous Claims, **characterized in that** the removable mini tank structure (9) comprises a lower opening (30) comprising a gasket element (31).

6. Nasal nebulizer according to one of the previous Claims, **characterized in that** the removable mini tank structure (9) comprises connecting parts (32) arranged for holding the mini tank structure (9) in a predefined position into the main tank (5).

7. Nasal nebulizer according to one of the previous Claims, **characterized in that** the removable mini tank structure (9) comprises connecting parts (32) act as abutments cooperating with the circular end surface (33) of the main tank structure (5) so as to maintain the mini tank structure (9) into the main tank (5) in said predefined position, preferably the connecting parts (32) comprise an external collar projecting outwardly.

8. Nasal nebulizer according to one of the previous Claims, **characterized in that** the removable mini tank structure (9), the main tank structure (5) and the internal conduct (17) projecting upwardly into the mini tank structure (9) are coaxially arranged.

9. Nasal nebulizer according to one of the previous Claims, **characterized in that** it further comprises a mini internal cylinder (10) arranged so as to form a sleeve around the internal conduct (17) of the main tank structure (5), said mini internal cylinder (10) comprising a skirt-like peripheral wall projecting downwardly into the mini tank structure (9) toward the bottom surface (9c) of said mini tank structure (9), and said mini internal cylinder (10) being radially separated from the internal conduct (17) by an intermediary spacing (35).

10. Nasal nebulizer according to one of the previous Claims, **characterized in that** it further comprises an inner bell-shaped nozzle (7) and an upper part (8) with a bell-shaped portion (11) coaxially arranged on the mini internal cylinder (10).

11. Nasal nebulizer according to one of the previous Claims, **characterized in that** the inner bell-shaped nozzle (7), the upper part (8) having a bell-shaped portion (11) and the mini internal cylinder (10) are removable/detachable.

12. Nasal nebulizer according to one of the previous Claim, **characterized in that** the internal conduct (17), the external peripheral wall (20) and the bottom surface (21) forming the main tank structure (5) are made one-piece, for example by moulding.

13. Kit comprising :
- a nasal nebulizer according to one of the previous Claims, and
- at least one internal cylinder part (6) conceived and designed for being arranged so as to form a sleeve around the internal conduct (17) of the main tank structure (5), said internal cylinder part (6) comprising a skirt-like peripheral wall projecting downwardly into the main tank structure (5) toward the bottom surface (21) of the storage compartment (19) of said main tank structure (5), and said internal cylinder part (6) being radially separated from the internal conduct (17) by an intermediary spacing (25).

## Patentansprüche

1. Nasalzerstäuber, Folgendes umfassend:
- einen Basisteil (1) mit einem Gaseinsatz (14) und einem internen Gasdurchlass (14'),
- eine Haupttankanordnung (5), getragen von einem Basisteil (1) und umfassend ein internes Fach (19), und
- eine interne Führung (17) in Fluidverbindung mit dem internen Gasdurchlass (14') des Basisteils (1), die sich nach oben in das interne Fach (19) der Haupttankanordnung (5) erstreckt,
**dadurch gekennzeichnet, dass** sie ferner eine ausbaubare Mini-Tankanordnung (9) umfasst, die in der Haupttankanordnung (5) angeordnet ist, und eine untere Öffnung (30) umfasst, wobei sich die interne Führung (17) durch die untere Öffnung (30) der Mini-Tankanordnung (9) nach oben erstreckt.

2. Nasalzerstäuber nach dem vorhergehenden Anspruch, **dadurch gekennzeichnet, dass** die Mini-Tankanordnung (9) eine Becherform aufweist.

3. Nasalzerstäuber nach einem der vorhergehenden Ansprüche, **dadurch gekennzeichnet, dass** die Mini-Tankanordnung (9) eine interne Kammer (34) mit einem verringerten Volumen aufweist, das kleiner ist als das Volumen des internen Fachs (19) der Haupttankanordnung (5).

4. Nasalzerstäuber nach einem der vorhergehenden Ansprüche, **dadurch gekennzeichnet, dass**:
- die Mini-Tankanordnung (9) eine interne Kammer (34) mit einem verringerten Volumen aufweist, das kleiner ist als 5 ml, vorzugsweise höchstens 3 ml, und
- das interne Fach (19) der Haupttankanordnung (5) ein internes Volumen von wenigstens 10 ml aufweist, vorzugsweise wenigstens 15 ml.

5. Nasalzerstäuber nach einem der vorhergehenden Ansprüche, **dadurch gekennzeichnet, dass** die ausbaubare Mini-Tankanordnung (9) eine untere Öffnung (30) mit einem Dichtungselement (31) aufweist.

6. Nasalzerstäuber nach einem der vorhergehenden Ansprüche, **dadurch gekennzeichnet, dass** die ausbaubare Mini-Tankanordnung (9) Verbindungsteile (32) aufweist, die angeordnet sind, um die Mini-Tankanordnung (9) in einer vorbestimmten Position im Haupttank (5) zu halten.

7. Nasalzerstäuber nach einem der vorhergehenden Ansprüche, **dadurch gekennzeichnet, dass** die ausbaubare Mini-Tankanordnung (9) Verbindungsteile (32) umfasst, die als Anschläge wirken, welche mit der runden Endfläche (33) der Haupttankanordnung (5) zusammenwirken, um die Mini-Tankanordnung (9) in der vordefinierten Position im Haupttank (5) zu halten, wobei die Verbindungsteile (32) vorzugsweise einen nach außen vorstehenden externen Kragen aufweisen.

8. Nasalzerstäuber nach einem der vorhergehenden Ansprüche, **dadurch gekennzeichnet, dass** die ausbaubare Mini-Tankanordnung (9), die Haupttankanordnung (5) und die interne Führung (17), die sich nach oben in die Mini-Tankanordnung hinein erstreckt (9), koaxial angeordnet sind.

9. Nasalzerstäuber nach einem der vorhergehenden Ansprüche, **dadurch gekennzeichnet, dass** er ferner einen internen Mini-Zylinder (10) umfasst, der derart angeordnet ist, dass er eine Hülse um die interne Führung (17) der Haupttankanordnung (5) ausbildet, wobei der interne Mini-Zylinder (10) eine schürzenähnliche umlaufende Wand umfasst, die in Richtung der unteren Oberfläche (9c) der Mini-Tankanordnung (9) nach unten in die Mini-Tankanordnung (9) hinein hervorsteht, und wobei der interne Mini-Zylinder (10) durch einen Zwischendistanzhalter (35) radial von der internen Führung (17) beabstandet ist.

10. Nasalzerstäuber nach einem der vorhergehenden Ansprüche, **dadurch gekennzeichnet, dass** er ferner eine innere glockenförmige Düse (7) und einen oberen Teil (8) mit einem glockenförmigen Abschnitt (11) aufweist, der koaxial auf dem internen Mini-Zylinder (10) angeordnet ist.

11. Nasalzerstäuber nach einem der vorhergehenden Ansprüche, **dadurch gekennzeichnet, dass** die innere glockenförmige Düse (7), der obere Teil (8) mit einem glockenförmigen Abschnitt (11) und der interne Mini-Zylinder (10) ausbaubar / abnehmbar sind.

12. Nasalzerstäuber nach einem der vorhergehenden Ansprüche, **dadurch gekennzeichnet, dass** die interne Führung (17), die externe umlaufende Wand (20) und die untere Oberfläche (21), die die Haupttankanordnung (5) ausbilden, einstückig ausgebildet werden, zum Beispiel durch Spritzgießen.

13. Kit, Folgendes umfassend:
- einen Nasalzerstäuber nach einem der vorhergehenden Ansprüche, und
- wenigstens einen internen Zylinderteil (6), ausgelegt und gestaltet, um derart angeordnet zu werden, dass er eine Hülse um die interne Führung (17) der Haupttankanordnung (5) ausbildet, wobei der interne Zylinderteil (6) eine schürzenähnliche umlaufende Wand umfasst, die in Richtung der unteren Oberfläche (21) des Staufachs (19) der Haupttankanordnung (5) nach unten in die Haupttankanordnung (5) hervorsteht, und wobei der interne Zylinderteil (6) durch einen Zwischendistanzhalter (25) radial von der internen Führung (17) beabstandet ist.

## Revendications

1. Nébuliseur nasal comprenant :
- une partie de base (1) dotée d'une admission de gaz (14) et d'un passage de gaz interne (14'),
- une structure de réservoir principal (5) portée par la partie de base (1) et comprenant un compartiment interne (19), et
- un conduit interne (17) en communication fluidique avec le passage de gaz interne (14') de la partie de base (1), et dépassant vers le haut dans le compartiment interne (19) de la structure de réservoir principal (5),
**caractérisé en ce qu'**il comprend en outre une structure de mini réservoir amovible (9) agencée dans la structure de réservoir principal (5) et comprenant une ouverture inférieure (30), ledit conduit interne (17) dépassant vers le haut à travers ladite ouverture inférieure (30) de la structure de mini réservoir (9).

2. Nébuliseur nasal selon la revendication précédente, **caractérisé en ce que** la structure de mini réservoir (9) est en forme de coupelle.

3. Nébuliseur nasal selon l'une des revendications précédentes, **caractérisé en ce que** la structure de mini réservoir (9) comprend une chambre interne (34) ayant un volume réduit qui est inférieur au volume du compartiment interne (19) de la structure de réservoir principal (5).

4. Nébuliseur nasal selon l'une des revendications précédentes, **caractérisé en ce que** :
- la structure de mini réservoir (9) comprend une chambre interne (34) ayant un volume réduit qui est inférieur à 5 ml, de préférence de 3 ml ou moins, et
- le compartiment interne (19) de la structure de réservoir principal (5) a un volume interne qui est d'au moins 10 ml, de préférence de 5 ml ou plus.

5. Nébuliseur nasal selon l'une des revendications précédentes, **caractérisé en ce que** la structure de mini réservoir amovible (9) comprend une ouverture inférieure (30) comprenant un élément de joint (31).

6. Nébuliseur nasal selon l'une des revendications précédentes, **caractérisé en ce que** la structure de mini réservoir amovible (9) comprend des parties de raccordement (32) agencées pour contenir la structure de mini réservoir (9) dans une position prédéfinie dans le réservoir principal (5).

7. Nébuliseur nasal selon l'une des revendications précédentes, **caractérisé en ce que** la structure de mini réservoir amovible (9) comprend des parties de raccordement (32) servant de butées coopérant avec la surface d'extrémité circulaire (33) de la structure de réservoir principal (5) de façon à maintenir la structure de mini réservoir (9) dans le réservoir principal (5) dans ladite position prédéfinie, de préférence les parties de raccordement (32) comprennent un collier externe dépassant vers l'extérieur.

8. Nébuliseur nasal selon l'une des revendications précédentes, **caractérisé en ce que** la structure de mini réservoir amovible (9), la structure de réservoir principal (5) et le conduit interne (17) dépassant vers le haut dans la structure de mini réservoir (9) sont agencés coaxialement.

9. Nébuliseur nasal selon l'une des revendications précédentes, **caractérisé en ce qu'**il comprend en outre un mini cylindre interne (10) agencé de façon à former un manchon autour du conduit interne (17) de la structure de réservoir principal (5), ledit mini cylindre interne (10) comprenant une paroi périphérique de type jupe dépassant vers le bas dans la structure de mini réservoir (9) vers la surface de fond (9c) de ladite structure de mini réservoir (9), et ledit mini cylindre interne (10) étant séparé radialement du conduit interne (17) par un espacement intermédiaire (35).

10. Nébuliseur nasal selon l'une des revendications précédentes, **caractérisé en ce qu'**il comprend en outre une buse interne en forme de cloche (7) et une partie supérieure (8) dotée d'une portion en forme de cloche (11) agencées coaxialement sur le mini cylindre interne (10).

11. Nébuliseur nasal selon l'une des revendications précédentes, **caractérisé en ce que** la buse interne en forme de cloche (7), la partie supérieure (8) dotée d'une portion en forme de cloche (11) et le mini cylindre interne (10) sont amovibles/détachables.

12. Nébuliseur nasal selon l'une des revendications précédentes, **caractérisé en ce que** le conduit interne (17), la paroi périphérique externe (20) et la surface de fond (21) formant la structure de réservoir principal (5) sont réalisés d'un seul tenant, par exemple par moulage.

13. Nécessaire comprenant:
- un nébuliseur nasal selon l'une des revendications précédentes, et
- au moins une partie de cylindre interne (6) imaginée et conçue pour être agencée de façon à former un manchon autour du conduit interne (17) de la structure de réservoir principal (5), ladite partie de cylindre interne (6) comprenant une paroi périphérique de type jupe dépassant vers le bas dans la structure de réservoir principal (5) vers la surface de fond (21) du compartiment de stockage (19) de ladite structure de réservoir principal (5), et ladite partie de cylindre interne (6) étant séparée radialement du conduit interne (17) par un espacement intermédiaire (25).
